# EUROPEAN PATENT APPLICATION

(11) **EP 4 356 892 A1**
(43) Date of publication of application: **24.04.2024**
(21) Application number: 22202307.9
(22) Date of filing: 18.10.2022
(51) Int. Cl.: A61J 1/03, A61J 7/00

(54) **A MEDICAMENT DISPENSING ASSEMBLY AND KIT**

(71) Applicant: Medistac Inc. Limited, 1 Dublin (IE)
(72) Inventor: BEGGS, David, Dublin, D03 PW57 (IE); WALKIN, Kieran, Dublin, A96 Y276 (IE)
(74) Representative: Purdylucey Intellectual Property

(57) **Abstract**

A medicament dispensing kit comprising a booklet (100) comprising a front page (101) and a back page (102), the front page having an array of apertures (104) comprising at least two rows of seven apertures, in which the back page comprises a corresponding array of apertures (106) configured to register with the apertures of the front page when the booklet is closed, and a plurality of blister strips (200) including a first blister strip (200A) containing a first drug and a second blister strip (200B) containing a second drug. Each blister strip comprises a row of seven blisters (202) corresponding to the plurality of apertures (104, 106) in each row of apertures allowing the blister strip to nest snugly in a row of apertures of the front or back page of the booklet. Each column of apertures on the front and/or back page of the booklet is marked with an indicium (109) indicating a day of the week. The front page (101) of the booklet (100) usually comprises at least 4 rows of seven apertures (104).

## Description

### Field of the Invention

The present invention relates to a medicament dispensing assembly, a method of preparing a medicament dispensing assembly, and a method of dispensing drugs that employs a medicament dispensing assembly.

### Background to the Invention

Currently, the assembly of dispensing drugs in a pharmacy involves a pharmacist or pharmacist technician removing tablets from a blister pack counting out the required number of tablets for each drug for a given period such as a month and providing the drugs to the patient. This is time consuming and laborious, especially when a patient is taking a number of different medications. It is also prone to error as well as requiring an unnecessary amount of packaging. In the case of older patients taking a number of medications, the tablets are often provided in drug dispensing trays that have multiple individual containers for tablets, for example a weekly tray having a container for each day of the week or a four-week container having a container for a four-week period. Each container may contain multiple tablets, including tablets for each day of the week and tablets for specific dosing times. Preparation of this type of dispensing tray is time consuming and laborious, especially as tablets that are provided in a blister pack under controlled manufacturer conditions and then have to be removed from blisters in a pharmacy unnecessarily. This gives rise to enhanced chances of error and/or contamination.

Some pharmacies provide weekly or monthly medication for patients in the form of large tailor-made blister packs, with the tablets for each day of the week or month provided in an individual blister chamber. These blister packs are time consuming and laborious to prepare and add unnecessary costs to the dispensing process.

JP2012131564 describes a dispensing assembly for dispensing a single medication provided in a blister pack. The assembly comprises a month-per-page calendar, having the days of the month arranged in rows of seven (one row per week), and a row of apertures provided under each row of days for receipt of a 7-tablet blister pack. A number of 70-tablet blister strips of the medication is attached to a card in rows, and the card is placed in between the month page with the apertures and a backing page with corresponding apertures such that the blisters containing the drug project through the apertures. While this is a good method for dispensing a single drug on a monthly basis, it does not work for patients who are taking multiple drugs per day, or at specific times of each day. In addition, the use of the assembly is quite laborious insofar as it requires blister strips of seven tablets to be attached to a backing page in the correct orientation and spacing, and then the blister strip page to be sandwiched between two pages of the calendar with the blisters in register with the apertures.

It is an objective of the invention to overcome at least one of the above-referenced problems.

### Summary of the Invention

In a first aspect, the invention provides a medicament dispensing kit comprising:
a booklet comprising a front page and a backing page, the front page having at least one row of apertures comprising a plurality of apertures, and a back page having a corresponding row of apertures in register with the apertures of the front page when the booklet is closed; and
at least one blister strip containing a plurality of unit doses of a first drug, wherein the at least one blister strip comprises a row of blisters corresponding to the plurality of apertures in the at least one row of apertures allowing the blister strip to nest snugly in a row of apertures of the front or back page of the booklet.

In another aspect, the invention provides a medicament dispensing kit comprising:
a booklet comprising a front page and a backing page, the front page having an array of apertures comprising at least two rows of apertures, each row of apertures typically comprising a plurality of apertures, and a back page having a corresponding array of apertures in register with the apertures of the front page when the booklet is closed; and
a plurality of blister strips including a first blister strip containing a first drug and a second blister strip containing a second drug, wherein each blister strip comprises a row of blisters corresponding to the plurality of apertures in each row of apertures allowing the blister strip to nest snugly in a row of apertures of the front or back page of the booklet.

The kit of the invention can be provided to a pharmacy allowing a pharmacist to quickly and efficiently prepare a patient-specific medicament for dispensing of multiple drugs daily. As the kit employs blister strips of different drugs, each tailor made for the booklet of the kit, compiling a number of different blister strips into the same booklet is a relatively easy task and negates the need to de-blister medication packaged in a manufacturing plant. The patient is presented with a booklet comprising a regular array of blisters (each containing a medicament) with X columns (e.g., 7 columns each column corresponding to a day of the week) and Y rows (e.g., 4 rows corresponding to the four medicaments to be taken every day). The manufacture of the kit involves different drugs being purchased in bulk and repackaged into strips that are designed to nest in a row of apertures in the booklet.

Alternatively, the different drug may be purchased already packaged into blister strips. The booklets and the blister strips of medicaments are then supplied to a pharmacy allowing a pharmacist or pharmacist technician to prepare a patients specific medicament dispensing assembly suitable for a week or a longer period. The preparation time using the kit and assembly of the invention is of the order of 4 to 5 minutes compared to the current system which takes in excess of 15 minutes. At present no other pharmaceutically manufactured blisters are designed such to allow two different medications to be placed into a system of packaging with the net result of uniformity to the patient.

Generally, the spacing of the blisters in each blister strip is the same, allowing each blister strip to nest in a row of apertures in the booklet. The shape of the convex blister in the first and second blister strips may be the same or different, but they will be dimensioned so that they fit inside the apertures of the booklet. For example, if the first drug is a circular disk-shaped tablet, each blister in the strip may have a round dome shape. Likewise, if the first drug is a capsule, each blister in the strip for that drug may be capsule shaped.

In any embodiment, the blisters of each of the blister strips have the same dimensions (e.g., the same size and shape).

In any embodiment, all of the apertures in the booklet are the same size.

In any embodiment, the size of the blisters in the blister strips matches the size of the apertures on the front (and/or rear) page of the booklet.

In any embodiment, each row of apertures in the booklet contains 7 apertures. This arrangement provides a booklet suitable for a weekly drug dispensing assembly, with each column of blisters corresponding to a day of the week, and the number of rows per column corresponding to the number of different drugs to be taken on any given day.

In any embodiment, each column of blisters on the front (and/or back) page is marked with an indicium indicating a day of the week (for example "M", or "Mon" or "Monday").

In any embodiment, each row of apertures in the booklet contains 28, 30 or 31 rows of apertures. This arrangement provides a booklet suitable for a monthly drug dispensing assembly, with each column of blisters corresponding to a day of the month, and the number of rows per column corresponding to the number of different drugs to be taken on any given day.

In any embodiment, each column of blisters on the front (and/or back) page is marked with an indicium indicating a day of the month (for example 1, 2, 3 etc)

In any embodiment, the front page of the booklet comprises at least 2, 3, 4, 5, 6, 7 or 8 rows of apertures.

In any embodiment, one or more of the rows on the front (and/or rear) page is marked with an indicium indicating a time of the day, for example morning, lunchtime or evening. This is useful when a particular medicament is supposed to be taken at a specific time during the day, and when the patient has a medicament to take at one time of the day and another medicament to take at a second time of the day.

In any embodiment, the front page is hingedly attached to the back page.

In any embodiment, the booklet comprises a fastener for fastening the front page and back page together to clamp the blister strips in between. The fastener may be, for example, a Velcro fastener, or a button, or an adhesive strip.

In any embodiment, the kit comprises a third blister strip containing a third drug.

In any embodiment, the kit comprises a fourth blister strip containing a fourth drug.

In any embodiment, the kit comprises a fifth blister strip containing a fifth drug.

In any embodiment, the booklet is formed of corrugated cardboard. In any embodiment, the corrugated cardboard has a thickness of 0.5 to 2.0 mm.

In any embodiment, each blister strip in the kit comprises an electronically readable tag, such as for example a barcode, QR code, RFID tag, or any other type of machine-readable tag. In any embodiment, the electronically readable tag is positioned on the blister strip such that it can be scanned when the blister strip is nested in a row of apertures and sandwiched between the front and back pages of the booklet.

In any embodiment, each booklet comprises an electronically readable tag, such as a barcode, QR code, or an RFID tag.

In another aspect, the invention provides a medicament dispensing assembly comprising:
a booklet comprising a front page and a backing page, the front page having an array of apertures comprising at least two rows of apertures, each row of apertures typically comprising a plurality of apertures, and a back page having a corresponding array of apertures in register with the apertures of the front page when the booklet is closed; and
a plurality of blister strips including a first blister strip containing a first drug and a second blister strip containing a second drug, wherein each blister strip comprises a row of blisters corresponding to the plurality of apertures in each row of apertures allowing the blister strip to nest snugly in a row of apertures of the front or back page of the booklet.
wherein the front page and back page of the booklet abut in a closed configuration, with the first blister strip sandwiched between the front and back pages and nested in a first row of apertures of the booklet and the second blister strip mounted between the front and back pages and nested in a second row of apertures of the booklet.

In any embodiment, a third blister strip is sandwiched between the front and back pages and nested in a third row of apertures of the booklet.

In any embodiment, a fourth blister strip is sandwiched between the front and back pages and nested in a fourth row of apertures of the booklet.

In any embodiment, a fifth blister strip is sandwiched between the front and back pages and nested in a fifth row of apertures of the booklet.

In any embodiment, rows of apertures each contain seven apertures and the blister strips each contain seven blisters.

In another aspect, the invention provides a medicament dispensing system comprising:
a medicament dispensing assembly of the invention; and
a holder for the medicament dispensing assembly configured to alert a patient at specific times (e.g., dispensing times) to take a medicament from the medicament dispensing assembly.

In any embodiment, the medicament dispensing system comprises a plurality of medicament dispensing assemblies.

In any embodiment, the dispensing time may be once a day, or more than once a day in the case of patients who have dosage regimes that require dosage at different times of the day.

In any embodiment, the holder may comprise a processor coupled to a signal emitter. The holder may comprise the signal emitter, and or the holder may comprise a wireless transmitter configured to send a signal to remote signal emitter (for example a speaker, screen or vibration module of a remote device such as a mobile phone) to cause the device to emit a signal. The processor may be pre-programmed to cause the signal emitter to emit a signal at one or more predetermined times during the day. The signal emitter is generally configured to emit a signal that is easily detectable by a human, for example a loud noise, a light, a vibration, or a combination of one or more.

In any embodiment, the holder may comprise a sensor operatively coupled to the processor and configured to detect when a medicament dispensing assembly is removed from the holder. The processor may be configured to cause the signal emitter to emit a signal (continuously or intermittently) until the sensor detects that a medicament dispensing assembly has been removed from the housing.

In any embodiment, the sensor may also be configured to detect when a medicament (or medicaments) has been removed from medicament dispensing assembly. The sensor may comprise a sensing module to monitor the medicaments contained in the blister packs when the medicament dispensing assembly is housed within the holder. The sensing module may be a visual sensing module.

The medicament dispensing assembly (generally the booklet of the medicament dispensing assembly) typically includes a machine-readable tag comprising data identifying the medicaments contained within the assembly, and the patient's dosage regime for the medicaments.

The holder generally includes a reader for the machine-readable tag which is operatively coupled to the processor. The processor is configured to receive the data on the machine-readable tag, optionally store the data in a memory, and actuate the signal emitter based on the dosage regime data received from the machine-readable tag.

In another aspect, the invention provides a method of assembling a patient-specific medicament dispensing assembly, the method comprising the steps of:
providing a medicament dispensing kit according to the invention;
providing a medicament prescription for the patient;
nesting a blister strip containing a first medicament in a first row of apertures of the front page of the booklet;
nesting a blister strip containing a second medicament in a second row of apertures of the front page of the booklet; and
closing the booklet and fastening the front and back pages together.

In any embodiment, the method comprises:
nesting a blister strip containing a third medicament in a first row of apertures of the front page of the booklet; and
optionally nesting a blister strip containing a fourth medicament in a fourth row of apertures of the front page of the booklet; and
optionally nesting a blister strip containing a fifth medicament in a fifth row of apertures of the front page of the booklet.

In any embodiment, each blister strip contains a single row of seven blisters, and the front page of the booklet contains rows of seven apertures.

In any embodiment, the front page of the booklet contains at least 4 or 5 rows of apertures.

In another aspect, the invention provides a method of preparing a kit for making a drug dispensing assembly, the method comprising the steps of
providing a plurality of the blister strips, including a plurality of blister strips for a plurality of different drugs;
providing a plurality of booklets, each booklet comprising a front page and a backing page, the front page having an array of apertures comprising at least two rows of apertures, each row of apertures comprising a plurality of apertures corresponding to the number of blisters in the blister strips and dimensioned to allow a blister strip nest in the row of apertures , and a back page having a corresponding array of apertures in register with the apertures of the front page when the booklet is closed;
assembling a kit comprising the plurality of booklets and the plurality of blister strips; and
providing a customer with the kit.

In any embodiment, the method includes the initial steps of:
purchasing in bulk quantities a plurality of different drugs;
removing the drugs from their packaging;
repacking the drugs in blister strips having the same number of blisters in each strip.

In any embodiment, the method comprises purchasing in bulk quantities at least 10, 20, 30, 40, 50, 60, 70, 80, 90 or 100 different drugs.

In any embodiment, the blister strips comprise a row of 7 or 28 blisters containing a drug.

In any embodiment, the blister strip comprises a single row of blisters.

In any embodiment, the method comprises providing at least 500, 1000, 1500, 2000, 3000 the blister strips, including a plurality of blister strips for at least 10 different drugs;

In any embodiment, the number of apertures in the rows of apertures of the booklet match the number of blisters in the blister packs.

In any embodiment, the assembled order comprises a plurality of booklets and a plurality of blister strips, in which each booklet comprises seven apertures in each row of apertures and each blister strips contains a single row of seven blisters.

Other aspects and preferred embodiments of the invention are defined and described in the other claims set out below.

### Brief Description of the Figures

**FIG. 1** is a plan view of a booklet forming part of a medicament dispensing kit and assembly of the invention.
**FIG.2** is a perspective view of the booklet of Figure in a partly folded configuration.
**FIG. 3** is a perspective view from below of a blister strip forming part of a medicament dispensing kit and assembly of the invention.
**FIG. 4** is a side elevational view of the blister strip of Figure 3.

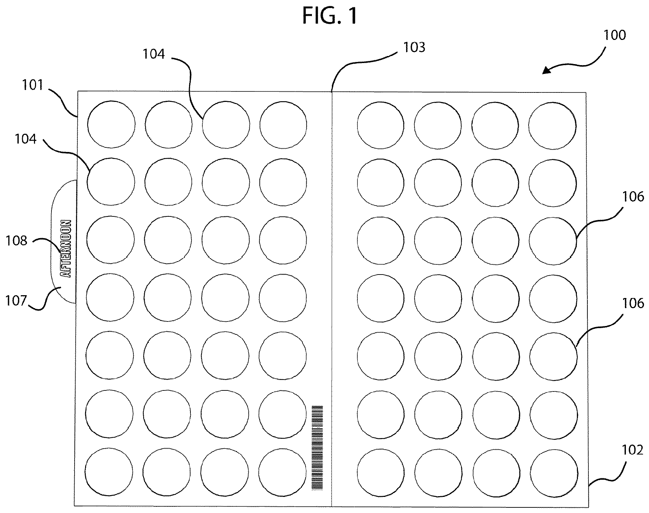

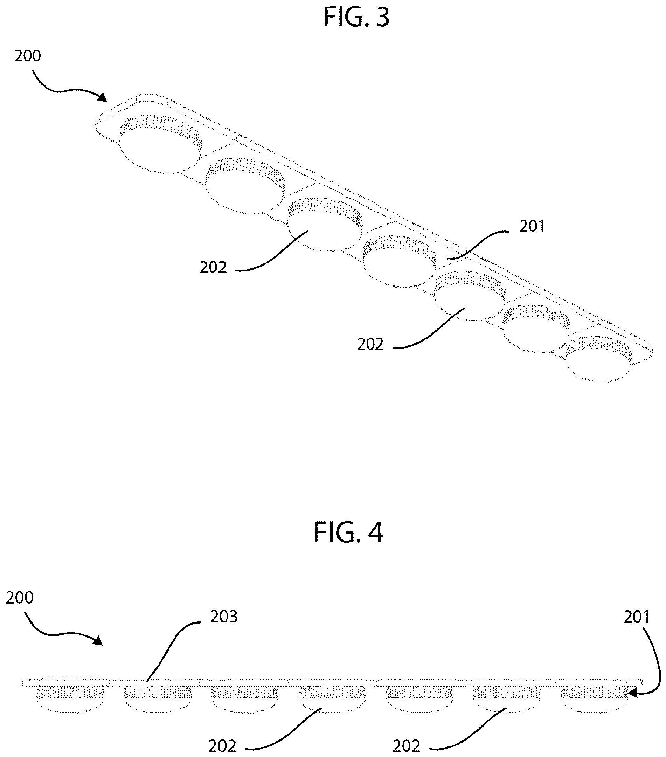

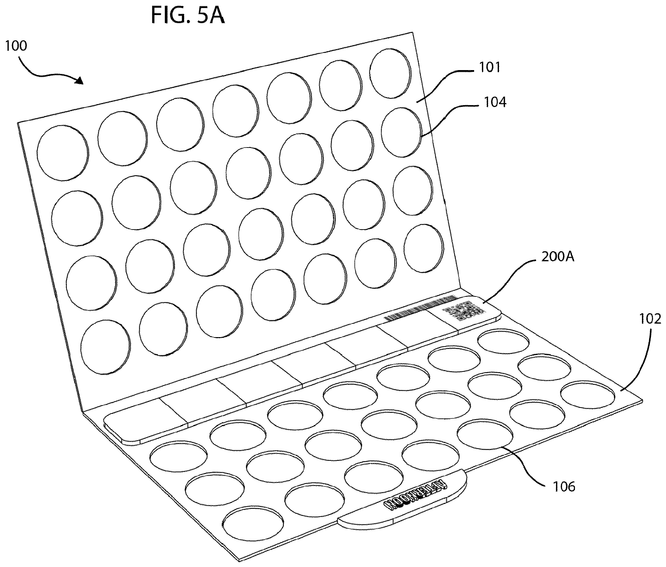

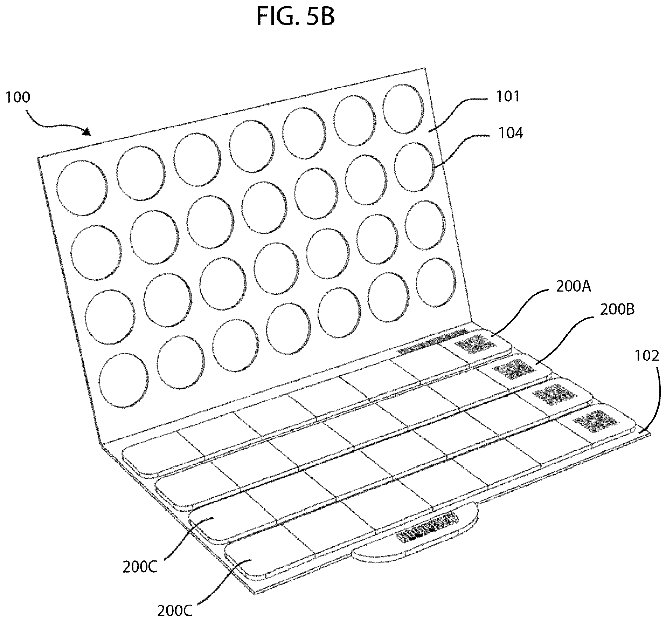

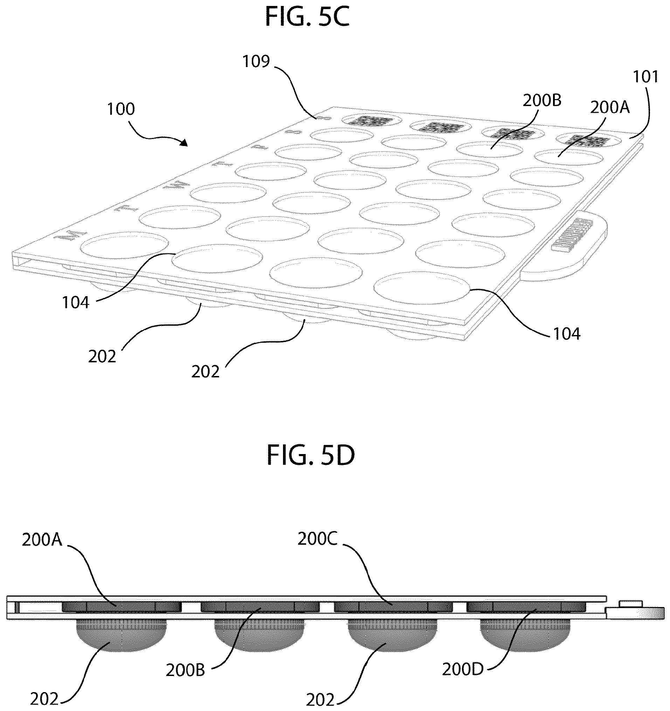

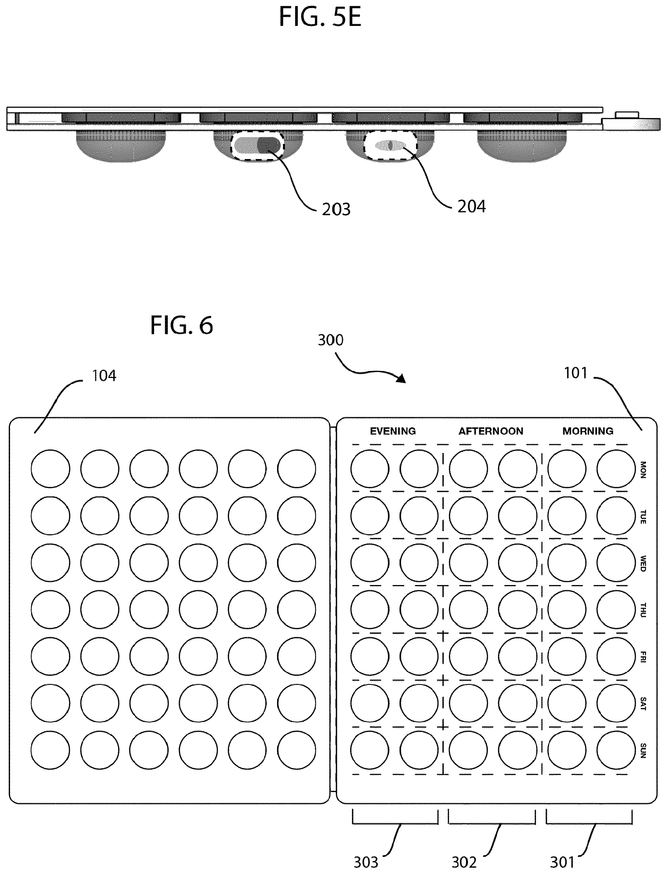

**FIG. 5** illustrates the assembly of a drug dispensing assembly of the invention, in which: **FIG. 5A** is a perspective view of the blister strip of Figure 3 being nested in a first row of apertures of the booklet of Figure 1; **FIG. 5B** is a perspective view of four blister strips, each containing a different drug, nested in rows of the booklet of Figure 1; **FIG. 5C** is a perspective view of the booklet in a closed configuration and four blister strips sandwiched between the front and back pages with the convex blisters projecting through the apertures; **FIG. 5D** is an end elevational view of the assembly of **FIG. 5C****.** **Fig. 5E** is the same as **FIG. 5D** but with cut-aways in the blister of the second and third blister strips illustrating a first type of drug 203 in the first blister strip and a second type of drug 204 in the second blister strip..
**FIG. 6** is a plan view of a booklet forming part of a medicament dispensing kit and assembly according to an alternative embodiment of the invention.
**FIG. 7A** is a block diagram illustrating a method of generating a medicament dispensing kit according to the invention suitable for supply to a pharmacy, in which the method includes the step of receiving different drugs in bulk and repackaging the drugs into blister strips dimensioned to nest in a booklet.
**FIG. 7B** is a block diagram illustrating an alternative method of generating a medicament dispensing kit according to the invention suitable for supply to a pharmacy, in which the drugs are provided pre-packaged in blister strips dimensioned to nest in a booklet.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "medicament" refers to a unit dose pharmaceutical such as a pill, tablet, lozenge or capsule that is suitable for being contained within a blister of a blister pack. The pharmaceutical may be any type of drug, for example an antibiotic, anti-fungal, anti-viral, anti-inflammatory, anti-depressant, anxiolytic, anti-psychotic, anti-cancer, painkiller, muscle relaxant, steroid, or antihistamine.

As used herein, the term "blister strip" refers to a packaging format commonly used for drugs that has a base layer formed of polymer or another material and containing a plurality of pockets or depressions (hereafter "blisters") that are dimensioned to receive a medicament in unit dose form and a cover layer formed of a frangible material such as a metallic foil that covers the base layer closing the pockets or in some embodiments a peelable layer that can be peeled back to open the pockets. To dispense a tablet a user presses the base layer of a pocket to push a drug out of the pocket through the metallic foil or, in the case of a peelable layer, peels back the layer to open one or more pockets The strip generally comprises a single row of blisters each containing a unit dose of medicaments, usually seven blisters.

As used herein, the term "booklet" refers to a container for a number of blister strips comprising a front page having an array of apertures comprising a plurality of rows of apertures and a back page generally having a corresponding array of apertures. Each row of apertures generally includes seven apertures, although booklets containing rows of apertures with more or less apertures are also envisaged. For example, a page of the booklet may have an elongated aperture configured to receive a blister strip without individual holes for the blisters. The front and back pages are generally similarly sized so that they can be arranged in an abutting relationship with the apertures of the front page in register with the apertures of the back page. The aperture on the back page allows a user to access the back of the blister to deform the blister and push the medicament out of the blister through the frangible front cover. The aperture in the front cover allows the medicament to be removed from the booklet. The pages are generally hingedly connected together. The booklet usually includes a fastening means to fasten the pages together in an abutting configuration. The booklet may be made of reinforced cardboard.

As used herein the term "medicament dispensing assembly" refers to a booklet containing one, and typically at least two, three or four or more blister strips nested in rows of apertures of the booklet and sandwiched between the front and back pages. It is the assembly that is provided to patients, especially patients that have complicated prescriptions comprising more than one medicament. Generally, the assembly comprises at least 2, 3, 4, 5, 6, 7 or 8 different medicaments. In some embodiments the assembly comprises medicaments that are to be taken at different times of the day. For example, a medicament that is to be taken in the morning and a medicament that is to be taken in the evening. The medicament dispensing assembly (generally the booklet) generally includes a machine-readable tag containing electronic data relating to the medicaments in the assembly, for example the identity of the medicaments, the dosage regime for each medicament, identity of the patient, contact details for the pharmacy.

As used herein the term "medicament dispensing kit" refers to a supply of booklets and blister strips that can be used in pharmacies to make up patient-specific medicament dispensing assemblies of the invention. Generally, the kit comprises blister strips of at least 20, 30, 40, 50, 60, 70, 80, 90, 100, 500, or 1000different drugs.

As used herein, the term "in bulk" refers to at least 1000 unit dose pharmaceuticals.

As used herein, the term "medicament dispensing system" refers to one or more medicament dispensing assemblies and a holder for the medicament dispensing assembly configured to alert a patient at specific times (e.g., dispensing times) to take a medicament from the medicament dispensing assembly. The holder may be configured to alert the patient when a medicament is supposed to be taken, to alert a patient is a wrong medicament is taken at a given time or alert the patient when a medicament is not taken at the correct time. The dispensing time may be once a day, or more than once a day in the case of patients who have dosage regimes that require dosage at different times of the day. The holder may have a processor coupled to a signal emitter. The signal emitter may be part of the holder or may be part of a remote device, in which case the holder comprises a wireless transmitter configured to allow the processor and remote signal emitter communicate. The transmitter may be a Wi-Fi or Bluetooth transmitter. The processor may be configured to be pre-programmed to cause the signal emitter to emit a signal at one or more predetermined times during the day. The signal emitter is configured to emit a signal that is easily detectable by a human, for example a load noise, a vibration, a light, or a combination of any. The holder may comprise a sensor operatively coupled to the processor and configured to detect when a medicament dispensing assembly is removed from the holder. The processor may be configured to cause the signal emitter to emit a signal (continuously or intermittently) until the sensor detects that a medicament dispensing assembly has been removed from the housing. The sensor may also be configured to detect when a medicament (or medicaments) has been removed from medicament dispensing assembly. The sensor may comprise a sensing module to monitor the medicaments contained in the blister packs when the medicament dispensing assembly is housed within the holder. The sensing module may be a visual sensing module. The medicament dispensing assembly (generally the booklet of the medicament dispensing assembly) typically includes a machine-readable tag comprising data identifying the medicaments contained within the assembly, and the patient's dosage regime for the medicaments. The holder generally includes a reader for the machine-readable tag which is operatively coupled to the processor. The processor is configured to receive the data on the machine-readable tag, optionally store the data in a memory, and actuate the signal emitter based on the dosage regime data received from the machine-readable tag.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

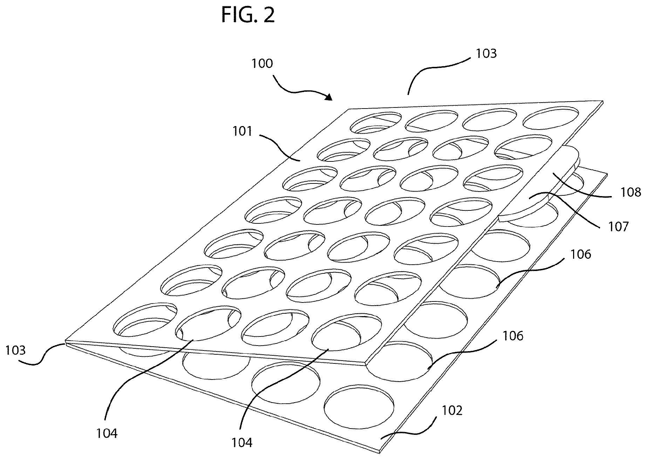

Referring to the drawings and initially to Figures 1 and 2 a booklet forming part of a medicament dispensing kit and assembly of the invention is illustrated, indicated generally by the reference numeral 100. The booklet is formed form reinforced cardboard having a thickness of about 1.2mm and comprises a front page 101 and a rear page 102 connected along an edge 103, each page having a width of about 11 cm and length of about 19 cm. The front page has a 7 x 4 array of apertures 104 with 4 rows and 7 columns. Each aperture 104 has a diameter of about 1.8 cm, and the apertures are equally spaced apart. The rear page has an identical array of identical apertures 106. The front page 101 also includes a tab 107 bearing a barcode 108.

As illustrated in Figure 2, the booklet is foldable along the edge 103 and can be fully folded to bring the pages 101 and 102 into an abutting configuration in which the apertures 104 of the front page 101 are in register with the apertures 106 of the rear page 102. The front of the front page 101 includes an indicium 109 at the top of each column of apertures providing an indication of the day of the week.

Figures 3 and 4 illustrate a blister strip 200 forming part of the assembly or kit of the invention and comprising a base layer 201 having a single row of seven equally spaced apart pockets 202 each of which contains a unit dose (not shown) of a specific medicament, and a top layer 203 adhered to the base layer to close the pockets. The pockets containing the unit dose medicaments are referred to herein as "blisters". Each blister has a diameter of about 1.7 cm (so that they fit snugly in the apertures 104 of the front page 101 of the booklet 100) and have the same spacing as the apertures allowing the strip 200 to nest in the row of apertures of the booklet. The base layer is formed of a hard polymer that keeps its shape while allowing a user to compress the wall of the pocket during ejection of a unit dose drug. The top layer is formed of a frangible metallic foil.

Figure 5 illustrates the steps of forming a medicament dispensing assembly according to the invention for a patient whose dosage regime comprises taking four different drugs each day. Figure 5A illustrates the booklet 100 in an open configuration and a first blister strip 200A containing a first drug mounted to the rear page 102 with the blisters 202 nested in a first row of apertures 106. Figure 5B shows the same booklet but with three additional blister strips 200B, 200C and 200D mounted to the other rows of apertures 106 in a similar manner. Figure 5C illustrates the booklet after the pages 101 and 102 have been closed together securing the four blister strips in between the pages. Figure 5D is a side elevational view of the assembled medicament dispensing assembly of Figure 5C showing how the blisters 202 project through the apertures 106 of the rear page and project proud of the surface of the rear page enabling the blisters to be pressed in to release a unit dose medicament. Figure 5E is the same as Figure 5D but with cut-aways in the blister of the second and third blister strips illustrating a first type of drug 203 in the first blister strip and a second type of drug 204 in the second blister strip.

The embodiment described above is an assembly for one week's medication. However, assemblies suitable for dispensing multiple medicaments to a patient over a longer period of time, for example a fortnight or one month are also envisaged and generally will employ longer strips with more blisters and wider pages with rows of apertures containing more apertures.

In addition, the rows of the apertures in the arrays can be grouped into rows for specific times of the day. Figure 6 illustrates a booklet 300 forming a part of a medicament dispensing assembly of the invention in which parts described with reference to the previous embodiments are assigned the same reference numerals. In this embodiment, the booklet is for an assembly suitable for dispensing drugs for a patient who takes one or more drugs in the morning, one or more drugs at lunchtime, and one or more drugs at night-time. The rows of each page are grouped into two rows at the top 301, two in the middle 302, and two at the bottom of the page 303, and the groupings are marked "Morning", "Lunchtime" and "Night". Alternatively, stickers may be employed, on the booklet or the blister strips, to indicate information to the patient for example the time of day when the medicament is to be taken.

The drug dispensing assemblies of the invention are usually assembled in a pharmacy by a pharmacist. The pharmacy will have access to a medicament dispensing kit from which to assemble patient-specific assembly. The kit generally comprises blister strips for a large number of drugs, for example more than 50, 100, 150, or 200 different drugs. The kit may include 50 or 100 blister strips of each drug. The kit may include over 1000 booklets with rows of apertures designed to correspond to the number and size of blisters on the blister strips.

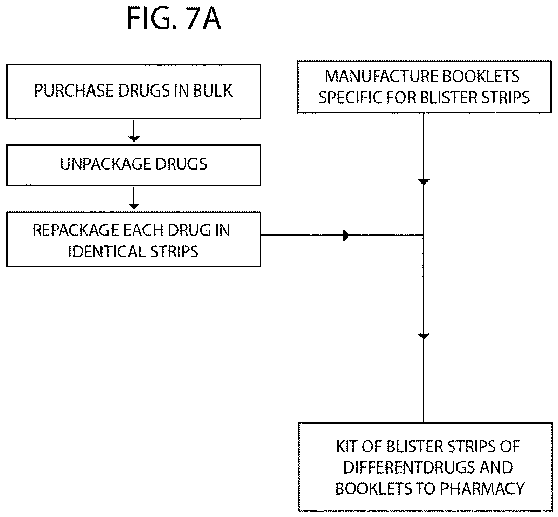

A block diagram 400 illustrating a method of making a kit of the invention is shown in Figure 7A. The method comprises a first step 410 of purchasing a plurality of different drugs in bulk number, for example 700 tables each of 20 different drugs. In a second step 420 the drugs are removed from their packaging. In a third step 430, 100 blister strips containing seven tablets of a drug are prepared for each of the 20 drugs, resulting in 2000 blister strips including 100 blister strips for each of the 20 drugs. Each blister strip is provided with a barcode containing identification data of the number and type of drug and the date of packaging. In a fourth step 440, booklets as described above are manufactured. In a fifth step 450, 1000 booklets are packaged together with the 2000 blister strips and the kit is delivered to a pharmacy where the kit can be used to assembly patient-specific medicament dispensing assemblies.

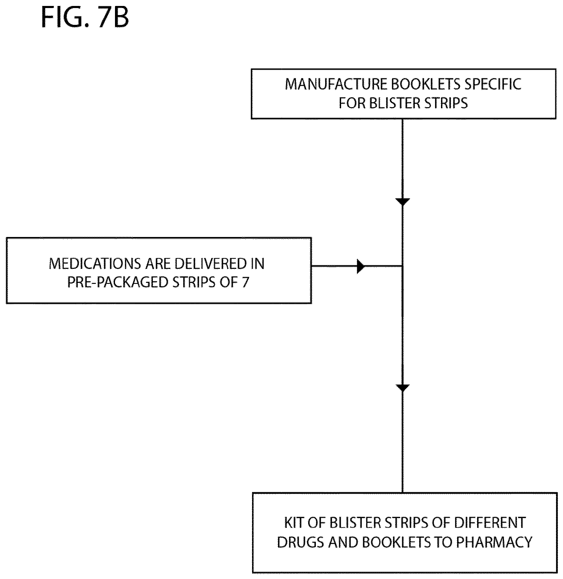

In a different embodiment, with reference to Figure 7B, the method is essentially the same as that described with reference to Figure 7A with the exception that drugs are provided by the drug manufacturer or distributor in custom-sized blister strips configured to fit in the booklets.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A medicament dispensing kit comprising:
a booklet (100) comprising a front page (101) and a back page (102), the front page having an array of apertures (104) comprising at least two rows of seven apertures, in which the back page comprises a corresponding array of apertures (106) configured to register with the apertures of the front page when the booklet is closed; and
a plurality of blister strips (200) including a first blister strip (200A) containing a first drug and a second blister strip (200B) containing a second drug, wherein each blister strip comprises a row of seven blisters (202) corresponding to the plurality of apertures (104, 106) in each row of apertures allowing the blister strip to nest snugly in a row of apertures of the front or back page of the booklet.
wherein each column of apertures on the front and/or back page of the booklet is marked with an indicium (109) indicating a day of the week.

2. A medicament dispensing kit according to Claim 1 in which the front page (101) of the booklet (100) comprises at least 4 rows of seven apertures (104).

3. A medicament dispensing kit according to Claim 1 or 2, including a third blister strip (200C) containing a third drug and a fourth blister strip (200D) containing a fourth drug, wherein each blister strip comprises a row of seven blisters (202) corresponding to the plurality of apertures (104, 106) in each row of apertures.

4. A medicament dispensing kit according to any preceding Claim, in which the front page (101) is hingedly attached to the back page (102).

5. A medicament dispensing kit according to any preceding Claim, in which the booklet (100) comprises a fastener for fastening the front page and back page together to clamp the pages together with the blister strips in between.

6. A medicament dispensing kit according to any preceding Claim, in which one or more of the rows on one of the pages is marked with a first indicia indicating a first time of the day and one or more of the other rows on the same page is marked with a second indicia indicating a different time of the day.

7. A medicament dispensing kit according to any preceding Claim, in which the kit comprises a third blister strip containing a third drug.

8. A medicament dispensing kit according to Claim 7, in which the kit comprises a fourth blister strip containing a fourth drug.

9. A medicament dispensing kit according to Claim 8, in which the kit comprises a fifth blister strip containing a fifth drug.

10. A medicament dispensing kit according to any preceding Claim, in which each blister strip in the kit comprises an electronically readable tag that is positioned on the blister strip such that it can be scanned when the blister strip is nested in a row of apertures and sandwiched between the front and back pages of the booklet, and in which each booklet comprises an electronically readable tag.

11. A medicament dispensing assembly assembled from a medicament dispensing kit of any of Claims 1 to 10, the medicament dispensing assembly comprising:
a booklet comprising a front page and a backing page, the front page having an array of apertures comprising at least two rows of seven apertures, and a back page having a corresponding array of apertures in register with the apertures of the front page when the booklet is closed; and
a plurality of blister strips including a first blister strip containing a first drug and a second blister strip containing a second drug, wherein each blister strip comprises a row of blisters corresponding to the plurality of apertures in each row of apertures allowing the blister strip to nest snugly in a row of apertures of the front or back page of the booklet.
wherein the front page and back page of the booklet abut in a closed configuration, with the first blister strip sandwiched between the front and back pages and nested in a first row of apertures of the booklet and the second blister strip mounted between the front and back pages and nested in a second row of apertures of the booklet.

12. A medicament dispensing assembly according to Claim 11 including a third or fourth blister strip sandwiched between the front and back pages and nested in a third row of apertures of the booklet.

13. A medicament dispensing system comprising:
one or more medicament dispensing assemblies according to any of Claims 11 or 12; and
a holder for the medicament dispensing assembly configured to alert a patient at specific times to take a medicament from the medicament dispensing assembly, the holder comprising:
a housing configured to removably receive a medicament dispensing assembly;
a processor;
a signal emitter operatively coupled to the processor configured to emit a signal that is easily detectable; and
a sensor operatively coupled to the processor and configured to detect when a medicament dispensing assembly is removed from the holder.
wherein the processor is configured to cause the signal emitter to emit a signal (continuously or intermittently) until the sensor detects that a medicament dispensing assembly has been removed from the housing.

14. A medicament dispensing system according to Claim 13, wherein:
the booklet of the medicament dispensing assembly comprises a machine-readable tag comprising data identifying the medicaments contained within the assembly, and the patient's dosage regime for the medicaments; and
the holder comprises a reader for the machine readable tag which is operatively coupled to the processor, in which the processor is configured to receive the data on the machine-readable tag, optionally store the data in a memory, and actuate the signal emitter based on the dosage regime data received from the machine-readable tag.

15. A method of preparing a medicament dispensing kit of any of Claims 1 to 10, the method comprising the steps of
purchasing in bulk quantities a plurality of different medicaments in unit dose form;
removing the medicaments from their packaging;
repacking the medicaments in blister strips having a single row seven blisters in each strip;
providing a plurality of the blister strips, including a plurality of blister strips for a plurality of different medicaments;
providing a plurality of booklets, each booklet comprising a front page and a backing page, the front page having an array of apertures comprising at least two rows of apertures, each row of apertures comprising a plurality of apertures corresponding to the number of blisters in the blister strips and dimensioned to allow a blister strip nest in the row of apertures , and a back page having a corresponding array of apertures in register with the apertures of the front page when the booklet is closed;
assembling a kit comprising the plurality of booklets and the plurality of blister strips; and
optionally, providing a customer with the kit.
